# EUROPÄISCHE PATENTANMELDUNG

(11) **EP 2 332 588 A2**
(43) Veröffentlichungstag der Anmeldung: **15.06.2011**
(21) Anmeldenummer: 10191222.8
(22) Anmeldetag: 15.11.2010
(51) Int. Cl.: A61L 27/04, A61L 27/30, A61L 27/50, A61L 27/58

(54) **Biokorrodierbares Implantat mit korrosionshemmender Beschichtung**

(30) Priorität: 10.12.2009 US 285190 P
(71) Anmelder: Biotronik VI Patent AG, 6341 Baar (CH)
(72) Erfinder: Rzany, Alexander, 90449, Nürnberg (DE); Müller, Heinz, 91052, Erlangen (DE)
(74) Vertreter: Lindner-Vogt, Karin L.

(57) **Zusammenfassung**

Die Erfindung betrifft ein biokorrodierbares Implantat auf Basis von Magnesium oder einer biokorrodierbaren Magnesiumlegierung, dessen Oberfläche eine korrosionshemmende Beschichtung aufweist. Ferner betrifft die Erfindung ein dazugehöriges Herstellungsverfahren für die Beschichtung. Erfindungsgemäß wird ein Implantat bereitgestellt mit
(i) einem eine Basis bildenden Implantatwerkstoff aus Magnesium oder einer biokorrodierbaren Magnesiumlegierung;
(ii) einer die Basis bedeckenden Diffusionsschicht, die sich in ihrer Zusammensetzung vom Implantatwerkstoff unterscheidet sowie zumindest eines der metallischen Elemente des Implantatwerkstoffs und zumindest ein korrosionsinhibierendes Element ausgewählt aus der Gruppe Al, Zn, Ca, Si, In, Mn, Sc und RE enthält, wobei RE für Seltenerdenmetalle steht;
(iii) optional, einer die Diffusionsschicht bedeckenden Metallschicht aus zumindest einem der korrosionsinhibierenden Elemente ausgewählt aus der Gruppe Al, Zn, Ca, Si, In, Mn, Sc und RE; und
(iv) optional, einer die Diffusionsschicht oder Metallschicht bedeckenden passivierenden Schicht aus Metalloxiden, -nitriden oder -fluoriden von zumindest einem der korrosionsinhibierenden Elemente ausgewählt aus der Gruppe Al, Zn, Ca, Si, In, Mn, Sc und RE.

## Beschreibung

Die Erfindung betrifft ein biokorrodierbares Implantat auf Basis von Magnesium oder einer biokorrodierbaren Magnesiumlegierung, dessen Oberfläche eine korrosionshemmende Beschichtung aufweist. Ferner betrifft die Erfindung ein dazugehöriges Herstellungsverfahren für die Beschichtung.

### Technologischer Hintergrund und Stand der Technik

Unter Implantat wird allgemein jede medizinische Vorrichtung aus einem oder mehreren Werkstoffen verstanden, die absichtlich in den Körper eingebracht wird und entweder teilweise oder ganz von einer Epitheloberfläche bedeckt ist. Implantate lassen sich hinsichtlich der Verwendungsdauer in Temporär- und Permanentimplantate untergliedern. Temporärimplantate verbleiben für einen befristeten Zeitraum im Körper. Permanentimplantate sind für den dauerhaften Verbleib im Körper vorgesehen. Bei Implantaten kann ferner zwischen Prothesen und künstliche Organe unterschieden werden. Eine Prothese ist eine medizinische Vorrichtung, die Gliedmaßen, Organe oder Gewebe des Körpers ersetzt, während unter einem künstlichen Organ eine medizinische Vorrichtung verstanden wird, die teilweise oder ganz die Funktion eines Körperorgans ersetzt. Unter die genannten Definitionen fallen beispielsweise Implantate wie orthopädische oder osteosynthetische Implantate, Herzschrittmacher, Defibrillatoren und vaskuläre Implantate.

Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßem Zweck mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems in das implantiert wird. So treten relativ kurzfristig Reizungen und Entzündungen auf, die zu Gewebeveränderungen führen können. Mittel- bis langfristig sind Vergiftungen, Allergien oder gar Krebsbildung Folgen mangelnder Biokompatibilität.

Biologische Systeme reagieren auf Fremdkörper in Abhängigkeit der Eigenschaften des Werkstoffs oder Bauteils in verschiedener Weise. Entsprechend der Reaktion des Biosystems können die Implantatswerkstoffe in bioaktive, bionierte und degradierbare/resorbierbare Werkstoffe unterteilt werden.

Für die Zwecke der vorliegenden Erfindung sind lediglich metallische Implantatwerkstoffe von Interesse, die ganz oder in Teilen aus Magnesium oder biokorrodierbaren Magnesiumlegierungen bestehen und deren Anwendung beispielsweise in der Osteosynthese, Gelenkersatz, Dentalchirurgie und Gefäßchirurgie liegt.

Ein Problem des Einsatzes biokorrodierbarer Magnesiumlegierungen ist die rasche Degradation des Materials in physiologischer Umgebung. Sowohl die Grundlagen der Magnesiumkorrosion als auch eine große Anzahl von technischen Verfahren zur Verbesserung des Korrosionsverhaltens (im Sinne einer Verstärkung des Korrosionsschutzes) sind aus dem Stand der Technik bekannt. Bekannt ist beispielsweise, dass der Zusatz von Yttrium und/oder weiteren Seltenerdmetallen Magnesiumlegierungen einen leicht erhöhten Korrosionswiderstand in Meerwasser verleiht.

Ein Ansatzpunkt zur Verbesserung des Korrosionsverhaltens sieht vor, eine korrosionsschützende Schicht auf dem aus Magnesium oder einer Magnesiumlegierung bestehenden Formkörper zu erzeugen. Bekannte Verfahren zur Erzeugung einer korrosionsschützenden Schicht wurden bisher aus dem Gesichtspunkt eines technischen Einsatzes des Formkörpers - jedoch nicht medizintechnischen Einsatzes biokorrodierbarer Implantate in physiologischer Umgebung - entwickelt und optimiert. Diese bekannten Verfahren umfassen: das Aufbringen von Polymeren oder anorganischen Deckschichten, das Erzeugen einer Emaille, die chemische Konversion der Oberfläche, Heißgasoxidation, Anodisierung, Plasmaspritzen, Laserstrahl-Umschmelzen, PVD-Verfahren, Ionenimplantation oder Lackieren.

Herkömmliche technische Einsatzgebiete von Formkörpern aus Magnesiumlegierungen außerhalb der Medizintechnik erfordern in der Regel eine weitgehende Unterbindung korrosiver Prozesse. Dementsprechend ist die Zielstellung der meisten technischen Verfahren eine vollständige Eliminierung korrosiver Prozesse. Dagegen sollte die Zielstellung zur Verbesserung des Korrosionsverhaltens von biokorrodierbaren Magnesiumlegierungen nicht in der vollständigen Unterbindung, sondern nur in der Hemmung bzw. der zeitlichen Verzögerung korrosiver Prozesse liegen. Schon aus diesem Grunde eignen sich die meisten bekannten Verfahren zur Erzeugung von Korrosionsschutzschichten auf Magnesium nicht. Ferner müssen für einen medizintechnischen Einsatz auch toxikologische Aspekte berücksichtigt werden. Des Weiteren sind korrosive Prozesse stark von dem Medium abhängig, in dem sie ablaufen, und daher dürfte eine Übertragbarkeit der unter herkömmlichen Umweltbedingungen auf technischem Gebiet gewonnenen Erkenntnisse zum Korrosionsschutz auf die Prozesse in physiologischer Umgebung nicht uneingeschränkt möglich sein. Schließlich dürfen bei einer Vielzahl von medizinischen Implantaten auch die der Korrosion zugrunde liegenden Mechanismen von üblichen technischen Anwendungen des Werkstoffs abweichen. So werden beispielsweise Stents, chirurgisches Material oder Clips im Gebrauch mechanisch verformt, sodass der Teilprozess der Spannungsrisskorrosion beim Abbau dieser Formkörper erhebliche Bedeutung haben dürfte.

Der Grundkörper einiger Implantate, wie insbesondere von Stents, wird im Gebrauch lokal unterschiedlich starken plastischen Verformungen unterworfen. Konventionelle Verfahren zur Hemmung der Korrosion, wie beispielsweise die Generierung einer dichten Magnesiumoxid-Deckschicht, sind hier nicht zielführend. Die keramischen Eigenschaften einer solchen Deckschicht würden zu einem lokalen Abplatzen der Deckschicht führen. Die Korrosion fände damit in unkontrollierter Weise statt und es bestünde insbesondere die Gefahr, dass in den mechanisch besonders beanspruchten Bereichen des Implantats die Korrosion forciert ist.

Der vorliegenden Erfindung liegt daher die Aufgabe zugrunde, eines oder mehrere der geschilderten Probleme zu beheben oder zumindest zu mindern. Insbesondere soll die Beschichtung ein hohes Haftungsvermögen besitzen und dennoch eine hinreichende Verformbarkeit gewährleisten.

### Erfindungsgemäße Lösung

Ein erster Aspekt der Erfindung liegt in der Bereitstellung eines Implantats mit
(i) einem eine Basis bildenden Implantatwerkstoff aus Magnesium oder einer biokorrodierbaren Magnesiumlegierung;
(ii) einer die Basis bedeckenden Diffusionsschicht, die sich in ihrer Zusammensetzung vom Implantatwerkstoff unterscheidet sowie zumindest eines der metallischen Elemente des Implantatwerkstoffs und zumindest ein korrosionsinhibierendes Element ausgewählt aus der Gruppe Al, Zn, Ca, Si, In, Mn, Sc und RE enthält, wobei RE für Seltenerdenmetalle steht;
(iii) optional, einer die Diffusionsschicht bedeckenden Metallschicht aus zumindest einem der korrosionsinhibierenden Elemente ausgewählt aus der Gruppe Al, Zn, Ca, Si, In, Mn, Sc und RE; und
(iv) optional, einer die Diffusionsschicht oder Metallschicht bedeckenden passivierenden Schicht aus Metalloxiden, -nitriden oder -fluoriden von zumindest einem der korrosionsinhibierenden Elemente ausgewählt aus der Gruppe Al, Zn, Ca, Si, In, Mn, Sc und RE.

Die erfindungsgemäße Diffusionsschicht entsteht, wie weiter unten noch näher ausgeführt, durch thermische Behandlung eines mit den korrosionsinhibierenden Elementen bedeckten Implantatsgrundkörpers aus Magnesium bzw. einer biokorrodierbaren Magnesiumlegierung. Die Diffusionsschicht unterscheidet sich in ihrer Zusammensetzung vom Implantatswerkstoff, beinhaltet aufgrund des Herstellungsprozesses jedoch zumindest eines der metallischen Elemente des Implantatswerkstoffs; in der Regel ist dies zumindest Magnesium. Ferner enthält die Diffusionsschicht ein oder mehrere der Elemente Al, Zn, Ca, Si, In, Mn, Sc und RE. Die metallische Diffusionsschicht kann darüber hinaus weitere Elemente enthalten. Die metallische Diffusionsschicht weist zumindest nahe der Basis des Implantats eine dem Basismaterial vergleichsweise Duktilität auf, sodass Verformungen des Implantats, wie sie beispielsweise im Zuge der Dilatation eines Stents auftreten, ohne Rissbildung und Abplatzen der korrosionshemmende Beschichtung erfolgen können. Eine Schichtdicke der Diffusionsschicht sollte so vorgegeben werden, dass beim Auftreten von Rissen oder bei dem, bei plastischen Verformungen nicht vermeidbaren, Austritt von sogenannten Gleitstufen, diese Beschädigungen der ursprünglichen Oberfläche nicht bis in das Grundmaterial reichen, sondern nur in einen Bereich, in dem das korrosionshemmende Element noch angereichert ist und so ein lokaler Korrosionsangriff vermieden wird.

Unter der Sammelbezeichnung "Seltenerdmetalle" werden vorliegend Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Als "biokorrodierbar" im Sinne der Erfindung werden Magnesiumlegierungen bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist. Die Magnesiumlegierung wird vorliegend als ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%.

Die Magnesiumlegierung ist so in ihrer Zusammensetzung zu wählen, dass sie biokorrodierbar ist. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaC12 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Die erfindungsgemäße Diffusionsschicht kann zusätzlich mit einer Metallschicht bedeckt sein, die zumindest eines der genannten korrosionsinhibierenden Elemente beinhaltet.

Eine Konzentration des korrosionsinhibierenden Elements in der Diffusionsschicht fällt vorzugsweise von einer Außenseite des Implantats hin zur Basis ab. Hierdurch wird erreicht, dass sich die Materialeigenschaften kontinuierlich angleichen, sodass ein sehr hohes Haftungsvermögen sicher gestellt ist.

Denkbar ist auch, die Diffusionsschicht so auszulegen, dass ein oder mehrere korrosionsinhibierende Elemente einem vorgebbaren Konzentrationsprofil über die Dicke der Diffusionsschicht folgen. Dies kann zum Beispiel derart geschehen, dass die Schritte (ii) und (iii) mit verschiedenen korrosionsinhibierenden Elementen und/oder unter verschiedenen Bedingungen (insbesondere Temperatur und Dauer der Behandlung) wiederholt werden.

Gemäß einer ersten bevorzugten Variante ist das korrosionsinhibierende Element ausgewählt aus der Gruppe Zn, Ca, In, Mn, Sc und RE; die genannten Elemente sind biokorrodierbar. Die Diffusionsschicht weist vorzugsweise eine Schichtdicke von 0.05 µm bis 20 µm auf. Ergänzend oder unabhängig davon kann die optionale Metallschicht eine Schichtdicke von 0.05 µm bis 20 µm aufweisen. Durch Einsatz der genannten biokorrodierbaren Elemente sind demnach relativ hohe Schichtdicken für Diffusionsschicht und/oder Metallschicht realisierbar, was in ausgewählten Anwendungsbereichen von Vorteil sein kann.

Nach einer alternativen, zweiten bevorzugten Variante ist das korrosionsinhibierende Element Al und/oder Si, insbesondere Al. Al und Si sind nicht biokorrodierbar und entsprechend ist eine Schichtdicke der Diffusionsschicht so gering auszulegen, dass dennoch ein allmählicher Abbau des Implantats möglich ist. Vorzugsweise weist die Diffusionsschicht eine Schichtdicke im Bereich von 0.01 µm bis 5 µm auf. Die ggf. zusätzlich noch vorhandene Metallschicht aus Al und/oder Si weist vorzugsweise eine Schichtdicke im Bereich von 0.01 µm bis 2 µm auf.

Die erfindungsgemäße Diffusionsschicht beziehungsweise auch die optionale Metallschicht kann neben den korrosionsinhibierenden Elementen weitere Elemente aufweisen, insbesondere Elemente mit pharmakologischer Wirkung. Diese Elemente umfassen vorzugsweise As, Se, Co, Cu und Ag.

Die erfindungsgemäße Diffusionsschicht beziehungsweise die optionale Metallschicht kann ferner mit einer passivierenden Schicht aus Metalloxiden, -nitriden oder -fluoriden von zumindest einem der korrosionsinhibierenden Elemente ausgewählt aus der Gruppe Al, Zn, Ca, Si, In, Mn, Sc und RE bedeckt sein.

Die Erfindung kann Anwendung bei allen Implantaten finden, die aus Magnesium oder biokorrodierbaren Magnesiumlegierungen bestehen oder die zumindest Bauteile aus diesem Implantatwerkstoff aufweisen, die nach der Implantation mit dem umgebenden Gewebe in Kontakt stehen. Besonders bevorzugt ist das Implantat ein vaskuläres, orthopädisches oder osteosynthetisches Implantat. Ganz besonders bevorzugt ist das Implantat ein Stent.

Ein weiterer Aspekt der Erfindung liegt in der Bereitstellung eines Verfahrens zur Oberflächenmodifikation eines Implantats, d.h. eines Verfahrens zur Herstellung der vorgenannten Diffusionsschicht. Das erfindungsgemäße Verfahren umfasst die Schritte:
(i) Bereitstellen eines unbehandelten Implantats mit einem eine Basis bildenden Implantatwerkstoff aus Magnesium oder einer biokorrodierbaren Magnesiumlegierung;
(ii) Kontaktieren der Oberfläche der Basis mit einem korrosionsinhibierenden Element ausgewählt aus der Gruppe Al, Zn, Ca, Si, In, Mn, Sc und RE, wobei RE für Seltenerdenmetalle steht; und
(iii) gleichzeitig oder im Anschluss an Schritt (ii), thermische Behandlung des Implantats zumindest im Bereich der Kontaktfläche unter Ausbildung einer die Basis bedeckenden Diffusionsschicht, die zumindest eines der metallischen Elemente des Implantatwerkstoffs und zumindest ein korrosionsinhibierendes Element ausgewählt aus der Gruppe Al, Zn, Ca, Si, In, Mn, Sc und RE enthält.

Nach dem erfindungsgemäßen Verfahren wird demnach die Oberfläche des aus Magnesium oder der biokorrodierbaren Magnesiumlegierung bestehenden Implantats mit dem korrosionsinhibierenden Elementen in Kontakt gebracht, d. h. in der Regel mit diesen Elementen beschichtet. Dies kann beispielsweise durch Beschichtungsverfahren, wie Dampfabscheidung, Elektrodenstrahlbeschichtung oder Sputtern erfolgen. In der Regel sollte eine möglichst homogene metallische Auftragung auf die Basis erfolgen.

Gleichzeitig mit der Beschichtung oder aber auch in einem sich anschließenden separatem Verfahrensschritt wird die Temperatur so eingestellt, dass sich eine Diffusionsschicht ausbilden kann. Die Temperatur ist dabei so vorzugeben, dass die mechanische Integrität des Grundkörpers nicht signifikant verschlechtert wird; die Temperatur liegt demnach zumindest unterhalb der Schmelztemperatur von Magnesium bzw. der eingesetzten biokorrodierbaren Magnesiumlegierung. Besonders vorteilhaft für das Verfahren ist somit der Einsatz von korrosionsinhibierenden Elementen wie Al, Zn, Ca, Si, In und Seltenerdmetallen, die eutektische Systeme mit Magnesium ausbilden. Bei nicht eutektischen Systemen, wie insbesondere der Verwendung von Mn oder Sc, bildet sich die Diffusionsschicht einzig durch thermisch induzierte Interdiffusionsprozesse.

Vorzugsweise wird der Schritt (iii) so durchgeführt, dass eine Konzentration des korrosionsinhibierenden Elements in der entstehenden Diffusionsschicht von einer Außenseite des Implantats hin zur Basis abnimmt.

Optional kann der ersten thermischen Behandlung eine zweite thermische Behandlung in sauerstoffreicher Atmosphäre folgen. Bei Einsatz von z.B. Aluminium kann auf diese Weise eine Verstärkung der Passivierung durch Oxidbildung an der Oberfläche erreicht werden.

Ferner kann in Anschluss an Schritt (ii) und gleichzeitig mit Schritt (iii) oder im Anschluss an Schritt (iii) eine chemische Reaktion der Oberfläche der Diffusionsschicht beziehungsweise Metallschicht mit O, N oder F unter Ausbildung einer das Implantat bedeckenden nichtmetallischen passivierenden Schicht durchgeführt werden. Die chemische Oberflächenbehandlung führt zur Ausbildung einer die Diffusionsschicht oder Metallschicht bedeckenden passivierenden Schicht aus Metalloxiden, -nitriden oder -fluoriden von zumindest einem der korrosionsinhibierenden Elemente ausgewählt aus der Gruppe Al, Zn, Ca, Si, In, Mn, Sc und RE. Hierdurch kann ein zusätzlicher passivierender Effekt erreicht werden.

Nachfolgend wird die Erfindung anhand eines Ausführungsbeispiels und dazugehöriger Zeichnungen näher erläutert. Die Figuren zeigen:
- Fig. 1:: einen Schnitt durch einen Probenkörper mit erfindungsgemäßer Diffusionsschicht;
- Fig. 2:: einen EDX-Scan desselben Probenkörpers;
- Fig. 3:: eine DSC-Kurve desselben Probekörpers; und
- Fig. 4:: eine schematische Darstellung möglicher Schichtabfolgen: (a) I+III: Grundkörper aus Legierung A mit Diffusionsschicht B(x)A(1-x) und optionaler Passivierung der Oberfläche (b) I+II+IV: Grundkörper aus Legierung A mit Diffusionsschicht B(x)A(1-x) und weiteren Diffusionsschichten C(y)(B(x)A(1-x))(1y) mit optionaler Passivierung der Oberfläche mit einer nichtmetallischen Deckschicht.

### Ausführungsbeispiel

Es wird ein Grundkörper aus einer biokorrodierbaren Magnesiumlegierung für einen Stent bereitgestellt. Die Magnesiumlegierung hat folgende Zusammensetzung:
3.9 Gew.% Y;
2.9 Gew.% weitere Seltenerdmetalle ohne Y;
0.4 Gew.% Zr; und
Rest Magnesium und herstellungsbedingten Verunreinigungen mit weniger als 0.1 Gew.%.

Der Grundkörper wird nachfolgend durch thermische Vakuumabscheidung mit einer etwa 2 µm bis 4 µm dicken Aluminiumschicht bedeckt. Anschließend wird der beschichtete Grundkörper 30 Min. in Luft auf 450°C aufgeheizt, wobei sich eine Diffusionsschicht ausbildet, die Aluminium und Magnesium enthält.

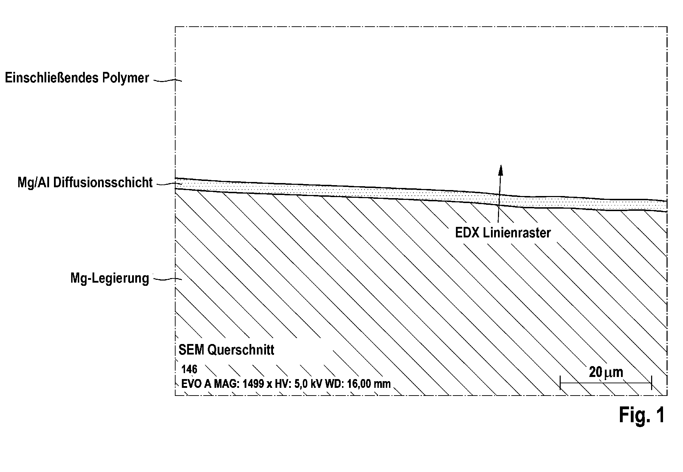

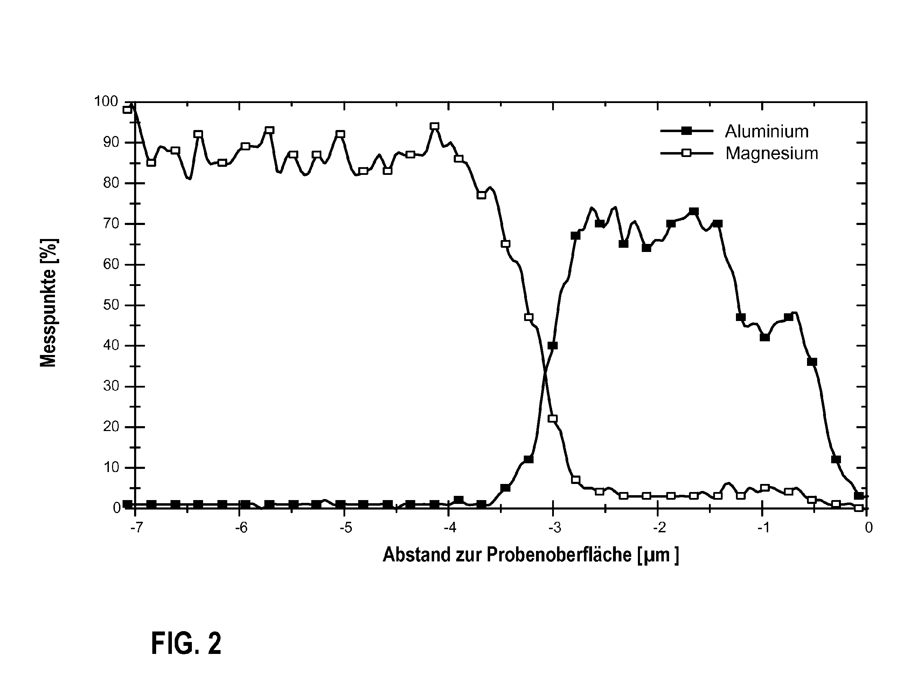

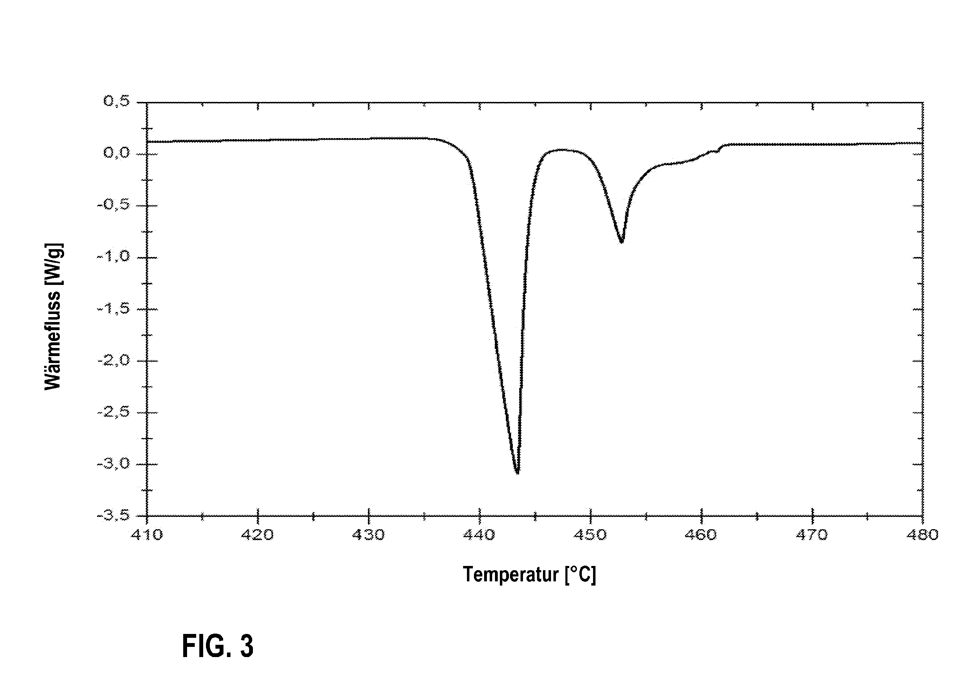

Figur 1 zeigt einen Schnitt durch den Probenkörper der Grenzflächen zwischen der Basis aus der biokorrodierbaren Magnesiumlegierung und der sich infolge der thermischen Behandlung gebildeten Diffusionsschicht. Eine elementaranalytische Untersuchung nach dem EDX-Verfahren belegt, dass sich an der Grenzfläche eine Al/Mg-Legierung bildet, deren Aluminiumgehalt nach Außen hin zunimmt (siehe Figur 2). Eine begleitende DSC-Untersuchung (siehe Figur 3) belegt die Bildung von zwei Eutektika bei 437°C und 450°C.

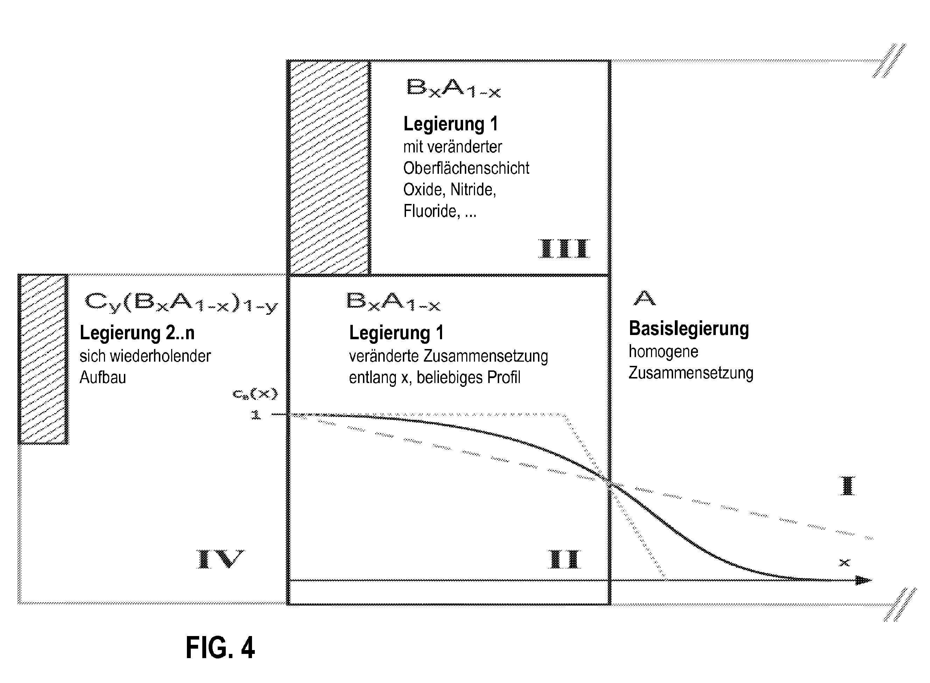

Figur 4 illustriert schematisch mögliche Schichtabfolgen: (a) I+III: Grundkörper aus Legierung A mit Diffusionsschicht B(x)A(1-x) und optionaler Passivierung der Oberfläche (schraffierter Bereich) (b) I+II+IV: Grundkörper aus Legierung A mit Diffusionsschicht B(x)A(1-x) und weiteren Diffusionsschichten C(y)(B(x)A(1-x))(1-y) mit optionaler Passivierung der Oberfläche mit einer nichtmetallischen Deckschicht (schraffierter Bereich).

## Patentansprüche

1. Implantat mit
(i) einem eine Basis bildenden Implantatwerkstoff aus Magnesium oder einer biokorrodierbaren Magnesiumlegierung;
(ii) einer die Basis bedeckenden Diffusionsschicht, die sich in ihrer Zusammensetzung vom Implantatwerkstoff unterscheidet sowie zumindest eines der metallischen Elemente des Implantatwerkstoffs und zumindest ein korrosionsinhibierendes Element ausgewählt aus der Gruppe Al, Zn, Ca, Si, In, Mn, Sc und RE enthält, wobei RE für Seltenerdenmetalle steht;
(iii) optional, einer die Diffusionsschicht bedeckenden Metallschicht aus zumindest einem der korrosionsinhibierenden Elemente ausgewählt aus der Gruppe Al, Zn, Ca, Si, In, Mn, Sc und RE; und
(iv) optional, einer die Diffusionsschicht oder Metallschicht bedeckenden passivierenden Schicht aus Metalloxiden, -nitriden oder -fluoriden von zumindest einem der korrosionsinhibierenden Elemente ausgewählt aus der Gruppe Al, Zn, Ca, Si, In, Mn, Sc und RE.

2. Implantat nach Anspruch 1, bei dem eine Konzentration des korrosionsinhibierenden Elements in der Diffusionsschicht von einer Außenseite des Implantats hin zur Basis abnimmt.

3. Implantat nach Anspruch 1 oder 2, bei dem das korrosionsinhibierende Element ausgewählt ist aus der Gruppe Zn, Ca, In, Mn, Sc und RE.

4. Implantat nach Anspruch 3, bei dem die Diffusionsschicht eine Schichtdicke im Bereich von 0.05 µm bis 20 µm aufweist.

5. Implantat nach Anspruch 3 oder 4, bei dem die Metallschicht eine Schichtdicke im Bereich von 0.05 µm bis 20 µm aufweist.

6. Implantat nach Anspruch 1 oder 2, bei dem das korrosionsinhibierende Element Al und/oder Si ist.

7. Implantat nach Anspruch 6, bei dem die Diffusionsschicht eine Schichtdicke im Bereich von 0.01 µm bis 5 µm aufweist.

8. Implantat nach Anspruch 6 oder 7, bei dem die Metallschicht eine Schichtdicke im Bereich von 0.01 µm bis 2 µm aufweist.

9. Implantat nach einem der vorhergehenden Ansprüche, bei dem das Implantat ein orthopädisches oder osteosynthetisches Implantat ist.

10. Implantat nach einem der Ansprüche 1 bis 8, bei dem das Implantat ein vaskuläres Implantat ist.

11. Verfahren zur Oberflächenmodifikation eines Implantats, umfassend die Schritte:
(i) Bereitstellen eines unbehandelten Implantats mit einem eine Basis bildenden Implantatwerkstoff aus Magnesium oder einer biokorrodierbaren Magnesiumlegierung;
(ii) Kontaktieren der Oberfläche der Basis mit einem korrosionsinhibierenden Element ausgewählt aus der Gruppe Al, Zn, Ca, Si, In, Mn, Sc und RE, wobei RE für Seltenerdenmetalle steht; und
(iii) gleichzeitig oder im Anschluss an Schritt (ii), thermische Behandlung des Implantats zumindest im Bereich der Kontaktfläche unter Ausbildung einer die Basis bedeckenden Diffusionsschicht, die zumindest eines der metallischen Elemente des Implantatwerkstoffs und zumindest ein korrosionsinhibierendes Element ausgewählt aus der Gruppe Al, Zn, Ca, Si, In, Mn, Sc und RE enthält.

12. Verfahren nach Anspruch 11, bei dem der Schritt (iii) so durchgeführt wird, dass eine Konzentration des korrosionsinhibierenden Elements in der entstehenden Diffusionsschicht von einer Außenseite des Implantats hin zur Basis abnimmt.

13. Verfahren nach Anspruch 11 oder 12, bei dem der Schritt (iii) so durchgeführt wird, dass die entstehende Diffusionsschicht von einer Metallschicht aus zumindest einem der korrosionsinhibierenden Elemente ausgewählt aus der Gruppe Al, Zn, Ca, Si, In, Mn, Sc und RE bedeckt ist.

14. Verfahren nach einem der Ansprüche 11 bis 13, bei dem in Anschluss an Schritt (ii) und gleichzeitig mit Schritt (iii) oder im Anschluss an Schritt (iii) eine chemische Reaktion der Oberfläche der Diffusionsschicht beziehungsweise Metallschicht mit O, N oder F unter Ausbildung einer das Implantat bedeckenden nichtmetallischen passivierenden Schicht durchgeführt wird.
